# EUROPEAN PATENT APPLICATION

(11) **EP 2 452 660 A1**
(43) Date of publication of application: **16.05.2012**
(21) Application number: 11172407.6
(22) Date of filing: 01.07.2011
(51) Int. Cl.: A61F 13/15, A61F 5/443, B65D 33/20

(54) **Disposable bag for biological waste**

(30) Priority: 11.11.2010 IT FG20100004 U; 11.11.2010 IT FG20100005 U; 11.11.2010 IT FG20100006 U
(71) Applicant: Angimedica S.r.l., 71121 Foggia (FG) (IT)
(72) Inventor: Folcando, Ettore, 71121 Foggia - FG (IT)
(74) Representative: Papa, Elisabetta

(57) **Abstract**

The present invention refers to a disposable bag for biological waste comprising a substance apt to absorb liquid and solid organic waste, characterized by a quick closure system. The disposable bag of the present invention is particularly useful for the disposal of biological fluids in hospital environments, and not only there.

## Description

### Field of the Invention

The present invention refers to a disposable bag for biological waste comprising a substance apt to absorb liquid and solid organic waste, characterized by a quick closure system. The disposable bag of the present invention is particularly useful for the disposal of biological fluids in hospital environments, and not only there.

### State of the Art

In the hospital care field, and not only there, there are problems regarding the correct collection of biological waste, like e.g. urine, feces and vomit.

In the state of the art suitable containers such as disposable bags and pouches, generally of polyethylene, are used for this purpose.

The fluid state of dejecta collected in those containers and the lack of quick closure systems causes leakage of part of the biological fluids before they are disposed of.

All such dejecta contain viable microorganisms or their toxins, considered for good reasons to be the cause of diseases in humans or other living organisms; therefore, the problem of providing containers for an effective disposal of biological fluids with closure systems such as to make a spreading of biological fluids in the surrounding environment unlikely is highly felt.

Object of the present invention is to solve the above-mentioned drawbacks by providing a disposable bag for biological fluids as substantially described in claim 1.

Additional features of the disposable bag for biological fluids of the invention at issue are defined in the corresponding dependent claims thereof.

The present invention, by overcoming the mentioned problems of the known art, entails several evident advantages.

In particular, the disposable bag of the present invention comprises a substance capable of absorbing dejecta, turning the same into a gel form, therefore more easily manageable, and a closure allowing operators in charge of waste disposal to quickly and efficiently close the bag, preventing any leakage of biological material in the surrounding environment, a possible cause of infections.

Still further advantages, as well as the features and operation steps of the present invention will be made evident in the following detailed description of some preferred embodiments thereof, given by way of example and not for limitative purposes. Reference will be made to the figures of the annexed drawings, wherein:
Figure 1 is a general perspective view showing, as a whole, an embodiment of the bag according to the present invention;
Figure 2 is a partially sectional perspective view showing, as a whole, the embodiment of the present invention of Figure 1;
Figure 3A is a longitudinal sectional view of the bag of Figure 1 and 2 along line A-A;
Figure 3B is a detail related to the closure system of the sectional view of Figure 3A;

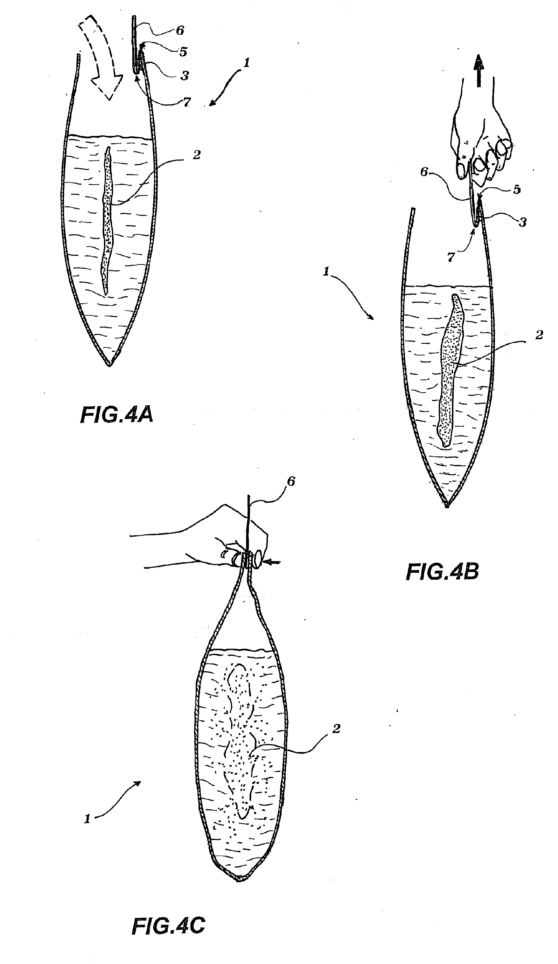

Figures 4A, 4B and 4C depict a sequence explaining the use of the disposable bag according to an embodiment, in particular of the closure system.

The present invention will hereinafter be described in detail making reference to the above-indicated figures.

### Detailed description of the invention

The present invention will hereinafter be described in detail making reference to the above-indicated figures.

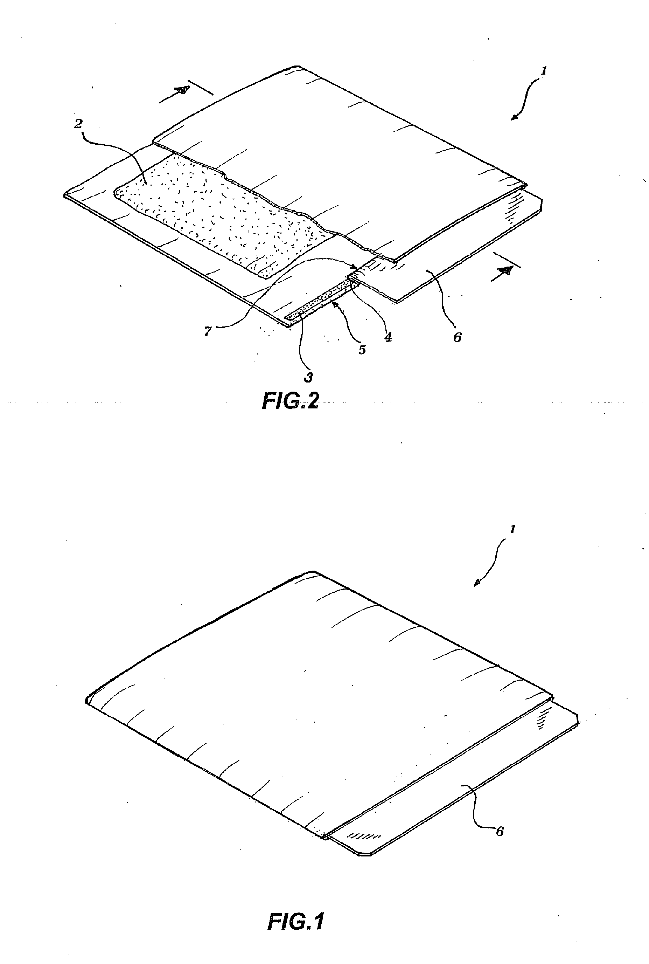

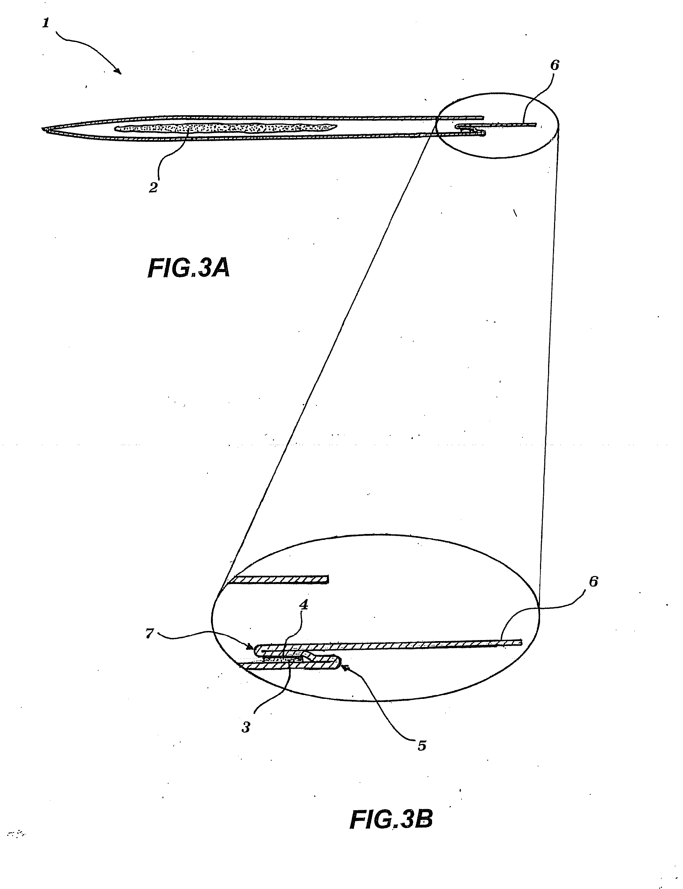

Referring initially to Figure 2 and to Figures 3A and 3B, these depict respectively a partially sectional and a longitudinal sectional perspective view showing, as a whole, a bag 1 according to the present invention, according to a preferred embodiment thereof.

The disposable bag of standard or biodegradable polyethylene 1 for biological fluids comprises therein a substance 2 apt to absorb liquid organic waste. Examples of suitable substances are superabsorbent powders having an absorbing capacity comprised between 60 and 500 times the weight of the fluids, like e.g. cross-linked sodium polyacrylate, available under the trade name of *Sgaiel.*

Advantageously, said substance 2 is contained in a water-soluble film; thus, the substance (e.g., in powder or granules) will be collected in the film without dispersing in the bag until the bag is filled with biological fluid.

The bag of the present invention is characterized by a closure system comprising:
- an adhesive element 3 internally fixed to said bag 1; preferably said adhesive element 3 consists of glue.
- a pre-flap 4 positioned so as to cover the adhesive element 3 and having a first edge 5 connected to an edge of the mouth of said bag 1;
- a flap 6 connected to a second edge 7 of said pre-flap 4 opposite to the first edge 5 and comprising a portion external to said bag 1.

The adhesive element 3 will preferably be fixed near and along the mouth of the bag 1. All systems for fixing the adhesive element 3 which are known to a person skilled in the art could be used, like e.g. the use of a biadhesive material.

The pre-flap 4 refers to an element positioned so as to cover the adhesive portion of the adhesive element 3 facing the inside of the bag 1 and connected by an edge 5 thereof to the edge of the mouth of the bag 1. The connection, as shown in Figure 2B, is on the same side of the bag 1 where the adhesive element 3 is fixed.

Instead, the flap 6 is connected to the edge of the pre-flap 4 (second edge 7) on the opposite side with respect to the first edge 5. This arrangement of the pre-flap 4 and the flap 6, on the side of the bag onto which the adhesive element 3 is fixed, forms the structure depicted in Figure 3B. The connections between the pre-flap 4 and the bag 1 and between the pre-flap 4 and the flap 6 may be obtained, e.g., by irreversibly sealing the various elements.

The flap 6 comprises a portion external to the bag 1, such as to enable the operator closing the bag to pull it; thus, the adhesive portion of the adhesive element 3 facing the inside of the bag will be exposed and free to adhere with the opposite side of the bag 1:
Then the bag 1, once the flap is pulled, can be closed by adhering the two sides of the bag.
Therefore, the closure system according to the present invention enables the operator to perform, after having filled the bag with biological fluids to be disposed of, a quick and effective closing.
In an embodiment, the bag 1 is made of polyethylene and manufactured by single folding of a polyethylene film and sealing of its sides.
The present invention has hereto been described with reference to some embodiments thereof. It is understood that other embodiments falling within the concept of the same invention might exist, all comprised within the protective scope of the claims hereinafter.

Dimensions and colors of the disposable bag of the present invention could be optimized according to the type of biological waste it shall contain. E.g., for urine it will preferably be in green, of dimensions of about 160x380 mm, for regurgitation in blue, of dimensions of about 250x250 mm and as bedpan cover in white, of dimensions of about 400x600 mm.

## Claims

1. A disposable bag (1) for biological waste, comprising therein a component made with a substance (2) apt to absorb liquid and/or solid organic waste, said bag being **characterized in that** it comprises a closure system having:
- an adhesive element (3) internally fixed to said bag (1);
- a pre-flap (4) positioned so as to cover the adhesive element (3) and having a first edge (5) connected to an edge of the mouth of said bag (1), and
- a flap (6) connected to a second edge (7) of said pre-flap (4) opposite to the first edge (5), and comprising a portion external to said bag (1).

2. The disposable bag (1) according to claim 1, wherein said substance (2) is contained in a water-soluble film.

3. The disposable bag (1) according to any one of the claims 1 to 2, wherein said substance is cross-linked sodium acrylate, provided with odorizing system.

4. The disposable bag (1) according to any one of claims 1 to 3, wherein said adhesive element consists of glue.

5. The disposable bag (1) according to any one of the claims 1 to 4, made of standard or biodegradable polyethylene.

6. The disposable bag (1) according to claim 5, wherein the bottom of said bag is obtained by single folding of a standard or biodegradable polyethylene film, and water-tight side sealing of one or more sides thereof.

7. The disposable bag (1) according to any one of the claims 1 to 6, wherein said biological waste is urine, feces or regurgitation.
